(19) Europäisches Patentamt
European Patent Office
Office européen des brevets

(11) EP 3 749 370 B1

(12) **EUROPÄISCHE PATENTSCHRIFT**

(45) Veröffentlichungstag und Bekanntmachung des Hinweises auf die Patenterteilung:
**07.05.2025 Patentblatt 2025/19**

(21) Anmeldenummer: **19702628.9**

(22) Anmeldetag: **07.02.2019**

(51) Internationale Patentklassifikation (IPC):
***A61K 47/32*** *(2006.01)* ***A61K 47/38*** *(2006.01)*
***A61K 9/14*** *(2006.01)* ***A61K 9/51*** *(2006.01)*
***A61K 31/4985*** *(2006.01)* ***A61K 9/00*** *(2006.01)*

(52) Gemeinsame Patentklassifikation (CPC):
**A61K 47/32; A61K 9/0056; A61K 9/006; A61K 9/10; A61K 9/2013; A61K 9/2027; A61K 9/2054; A61K 9/4858; A61K 9/4866; A61K 31/4985; A61K 47/20; A61K 47/38**

(86) Internationale Anmeldenummer:
**PCT/EP2019/052972**

(87) Internationale Veröffentlichungsnummer:
**WO 2019/154896 (15.08.2019 Gazette 2019/33)**

(54) **PHARMAZEUTISCHE FORMULIERUNGEN, VERFAHREN ZUM HERSTELLEN EINER PHARMAZEUTISCHEN FORMULIERUNG SOWIE EIN ARZNEIMITTEL UMFASSEND EINE SOLCHE**

PHARMACEUTICAL FORMULATIONS, METHOD FOR PRODUCING A PHARMACEUTICAL FORMULATION AND A MEDICAMENT COMPRISING SAME

FORMULATIONS PHARMACEUTIQUES, PROCÉDÉ DE PRÉPARATION D'UNE FORMULATION PHARMACEUTIQUE AINSI QUE MÉDICAMENT COMPRENANT UNE TELLE FORMULATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2018 EP 18155542**

(43) Veröffentlichungstag der Anmeldung:
**16.12.2020 Patentblatt 2020/51**

(73) Patentinhaber: **Smawa GmbH**
**97082 Würzburg (DE)**

(72) Erfinder:
- **ROEWER, Norbert**
  **97082 Würzburg (DE)**
- **BROSCHEIT, Jens**
  **97209 Würzburg (DE)**
- **STEER, Isabell**
  **45147 Essen (DE)**

(74) Vertreter: **Glawe, Delfs, Moll**
**Partnerschaft mbB**
**Postfach 13 03 91**
**20103 Hamburg (DE)**

(56) Entgegenhaltungen:
**EP-A1- 1 985 310**
**WO-A1-2014/003677**
**US-A1- 2007 104 792**
**US-A1- 2011 263 606**
**US-A1- 2015 359 735**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 749 370 B1

**Beschreibung**

Die Erfindung betrifft eine pharmazeutische Formulierung, eine Verfahren zum Herstellen einer pharmazeutischen Formulierung, eine pharmazeutische Formulierung, erhältlich durch eine solches Verfahren, und ein Arzneimittel umfassend eine pharmazeutische Formulierung.

Der Wirkstoff Tadalafil (IUPAC-Name: ((6R,12aR)-6-(1,3-Benzodioxol-5-yl)-2-methyl-1,2,3,4,6,7,12,12a-octahydropyrazino-[2',1':6,1]pyrido[3,4-b]indol-1,4-dion) gehört zur Gruppe der PDE-V (Phosphordiesterase V) Hemmer, die als orale Formulierung zur Behandlung von erektilen Dysfunktionen verwendet werden. Das Arzneimittel wird als Tablette zugeführt und intestinal, also über den Darm, aufgenommen. Die Aufnahme über den Darm erfolgt durch die Darmschleimhaut (intestinale Mucosa) des Dünndarms (Jejunum). Einerseits gehört Tadalafil zur Gruppe der "Small Molecules" (Molekulargewicht = 389 g/mol) mit lipophilen Eigenschaften (logP = +1,7) und kann als solches frei durch jedwede Membran diffundieren. Andererseits muss Tadalafil zur Membran gelangen und deshalb in dem dort vorherrschend wässrigen Medium lösbar sein. Tadalafil ist aufgrund seiner starken Neigung zur Kristallbildung in Wasser nur zu etwa 2-3,2 μg/ml löslich und gehört damit zur Gruppe der schwer in Wasser löslichen Substanzen.

Die Bioverfügbarkeit von oral zugeführtem Tadalafil ist direkt abhängig vom Grad der Löslichkeit in Wasser. Damit orale Formulierungen von Tadalafil trotz der Schwerlöslichkeit des Wirkstoffs eine ausreichend hohe Bioverfügbarkeit aufweisen, ist eine Verbesserung der Löslichkeit in Wasser notwendig. Ein wichtiger Schritt in diese Richtung ist die Aufbrechung der kristallinen Struktur des Feststoffes durch Mikronisierung, also die signifikante, mechanische Verkleinerung der mittleren Partikelgröße unter 200 μm. Eine klassische Methode zur Herstellung derart feiner Partikel ist das Zermahlen. Der Erfolg dieser Maßnahme ist allerdings abhängig davon, inwieweit es gelingt, eine Rekristallisation des mikronisierten Stoffes in wässriger Lösung zu verhindern.

Problematisch ist, dass die mikronisierten Teilchen zur Bildung von Agglomeraten neigen. Hierdurch entsteht eine ungleiche Verteilung der Partikelgrößen, die sich wiederum in unterschiedlichen Löslichkeiten wiederspiegelt. Eine durch den Vorgang des Zerkleinerns resultierende elektrostatische Aufladung des Wirkstoffs wirkt sich zudem negativ auf die Verarbeitbarkeit aus. Ein weiterer möglicher Nachteil ist die schlechte Fließfähigkeit des gemahlenen Wirkstoffes. Insbesondere, wenn Tabletten verpresst oder Kapseln befüllt werden sollen, werden daher weitere Verarbeitungsschritte, wie z. B. Granulierung, erforderlich. Pharmakokinetische Daten in der US-Patentanmeldung 2015/0359735 A1 offenbaren, dass bei der Anwendung eines Films, hergestellt aus einer Suspension umfassend mikronisiertes Tadalafil, Tensid und Cellulose-basierte Polymere, sich frühestens nach 2,50 Stunden der maximale Serumspiegel an Tadalafil-Wirkstoff ($t_{max}$) einstellt. Dieser lange Zeitraum ist unbefriedigend, insbesondere bei der Behandlung einer sexuellen Dysfunktion mit dem Tadalafil-Wirkstoff, beispielsweise im Rahmen der Behandlung einer akuten erektilen Dysfunktion, bei der ein schneller Behandlungserfolg einsetzen und/oder die prophylaktische Einnahme zur Sicherstellung einer bedarfsgerechten erektilen Funktion bereits im Vorfeld mit einem möglichst kurzem Latenz-Zeitfenster wünschenswert ist. "Latenz-Zeitfenster" ist dabei die Zeit, die von der Einnahme eines Arzneimittels bis zum Wirkungseintritt vergeht.

Der Erfindung liegt daher die Aufgabe zu Grunde, eine pharmazeutische Formulierung zu schaffen, die eine verbesserte Löslichkeit und Lösungsgeschwindigkeit von Tadalafil oder dessen Salz als Wirkstoff aufweist und somit zu einer gesteigerten pharmakologischen Effizienz durch einen schnelleren Wirkeintritt und eine erhöhte Bioverfügbarkeit führt.

Gelöst wird diese Aufgabe durch eine pharmazeutische Formulierung gemäß dem Hauptanspruch. Die Erfindung betrifft somit insbesondere eine pharmazeutische bukkale, sublinguale, gingivale oder intranasale Formulierung umfassend die folgenden Komponenten:

a) Tadalafil oder dessen Salz als Wirkstoff, wobei die mittlere Partikelgröße des Wirkstoffes in besagter Formulierung in einem Bereich von 8 bis 500 nm liegt,
b) ein Polymer, wobei besagtes Polymer Polyvinylpyrrolidon (PVP) und/oder Vinylpyrrolidon-Vinylacetat-Copolymer (KVA) ist, und
c) ein Tensid;

erhältlich durch ein Verfahren, in dem ein Zerkleinern von a) Tadalafil oder dessen Salz als Wirkstoff zumindest zusammen mit dem Polymer b) und dem Tensid c) erfolgt.

Weitere vorteilhafte Ausführungsformen finden sich in den Unteransprüchen.

Zunächst seien einige im Rahmen der Erfindung verwendete Begriffe erläutert.

Im Rahmen der nachfolgend näher beschriebenen Erfindung wird unter dem Begriff "mikronisierenden Polymer", zu denen auch vorgenannte Polymere PVP und KVA zuzurechnen sind, ein Polymer verstanden, das für die Zerkleinerung der Wirkstoffpartikel zusammen mit dem Tensid verwendet wird. Hiervon zu unterscheiden ist das im Rahme der Erfindung beschriebene "filmbildende Polymer", das zur Herstellung von Filmen eingesetzt wird. Es ist zu beachten, dass einigen wenigen Polymeren, wie insbesondere Hydroxypropylmethylcellulose, eine Doppelfunktion zukommen kann, so dass dieses im Rahmen der Erfindung als mikronisierendes Polymer und filmbildendes Polymer verwendet werden kann. Dieses ist allerdings nicht der Regelfall.

Die Erfindung hat erkannt, dass durch die Zugabe einer Kombination von mikronisierendem Polymer, insbesondere PVP und/oder KVA, und Tensid eine Stabilisierung der zerkleinerten Wirkstoffpartikel mit einer mittleren Partikelgröße von weit unter 500 nm, insbesondere unter 390 nm, problemlos möglich ist. Überraschenderweise treten die im Stand der Technik beschriebenen Probleme, wie Rekristallisation und die Bildung von Agglomeraten und/oder elektrostatische Aufladungen des Wirkstoffes, bei der erfindungsgemäßen pharmazeutischen Formulierung nicht auf. Denn während das mikronisierende Polymer erheblich zu einer sterischen Stabilisierung des Wirkstoffes beiträgt, wirkt die Zugabe des Tensids (SDS) einer ungewünschten elektrostatischen Aufladung der Wirkstoffpartikel entgegen. Die erfindungsgemäße pharmazeutische Formulierung ist daher in einfacher Weise handhabbar und zeigt daher eine verbesserte Lagerstabilität, eine deutlich erhöhte allgemeine Löslichkeit sowie eine höhere Lösungsgeschwindigkeit des Wirkstoffes. Die gesteigerte Löslichkeit des Wirkstoffes führt wiederum insbesondere bei der Verwendung von PVP und KVA zu einer überraschenden Verbesserung der pharmakologischen Effizienz durch einen schnelleren Wirkeintritt und eine erhöhte Bioverfügbarkeit. So sei an dieser Stelle bereits angemerkt, dass durch die spezielle Kombination der anspruchsgemäßen Komponenten a), b) und c) bereits nach weniger als 40 Minuten $t_{max}$, d.h. der Tadalafil-Spitzenplasmaspiegel ($c_{max}$) erreicht wurde, und damit um ein vielfaches schneller als bei der herkömmlichen Verabreichung einer handelsüblichen Cialis®-Tablette (s. Fig. 4) oder des in der US 2015/0359735 offenbarten Films umfassend neben dem Tadalalfil-Wirkstoff und Tensid Cellulose-basierte Polymere, jedoch nicht PVP oder KVA.

Im Rahmen der Erfindung ist es bevorzugt, dass die mittlere Partikelgröße des Wirkstoffes in einem Bereich von 10 bis 390 nm, noch bevorzugter in einem Bereich von 100 bis 390 nm, am meisten bevorzugt in einem Bereich von 200 bis 350 nm liegt. Bevorzugt ist es ferner, dass das neben den mikronisierenden Polymeren PVP und/oder KVA weitere Polymere umfasst sein können, ausgewählt aus Hydroxypropylmethylcellulose, Hydroxypropylcellulose, Methylcellulose und Gemischen hiervon. Weiter bevorzugt ist, dass das Tensid ein anionisches Tensid ist. Noch bevorzugter ist das anionische Tensid ausgewählt aus Alkylsulfaten, Alkylsulfonaten, Arylsulfaten, Arylsulfonaten und Gemischen hiervon. In einer besonders bevorzugten Ausführungsform ist das anionische Tensid Natriumdodecylsulfat.

Besonders bevorzugt sind pharmazeutische Formulierungen, bei denen neben der Tadalafil-Wirkstoffkomponente a) die Polymer- und Tensid-Komponenten wie folgt ausgewählt sind:

- besagte Polymer gemäß Komponente b) PVP und besagtes Tensid gemäß Konponente c) SDS, oder
- besagtes Polymer gemäß Komponente b) KVA und besagtes Tensid gemäß Komponente c) SDS, oder
- besagtes Polymer gemäß Komponente b) ein Gemisch aus PVP und KVA und besagtes Tensid gemäß Komponente c) SDS.

Zudem ist es bevorzugt, dass die pharmazeutische Formulierung ausgewählt ist aus der Gruppe bestehend aus

i) einem Film,
ii) einem Aerosol,
iii) einer wässrigen Suspension, Lösung, Tinktur, Creme, Paste, Lotion, Salbe, einem Gel, oder diese Formulierungen im Mundraum freisetzende Kapsel,
iv) einer Schmelz-, Lutsch- oder Bukkal-Tablette,

wobei vorgenannte Formulierungen vorzugsweise mukoadhäsive (schleimhauthaftende) Formulierungen sind.

Bei diesen pharmazeutischen Formulierungen wird der Tadalafil-Wirkstoff in der Formulierung über die Mucosa der Mundhöhle oder Nase den Blutkreislauf zur systemischen Wirkung zugeführt. Ein wesentlicher Vorteil liegt dabei insbesondere darin, dass der Wirkstoff nicht den Latenzzeit-verzögernden Umweg über den Magen-Darmtrakt nehmen muss, wie es bei herkömmlichen Cialis®-Tabletten der Fall ist und auch eine intravenöse Verabreichung vermieden werden kann, die zum einem unbequem ist und zum anderen bei unsachgemäßer Ausführung mit einer Reihe von unerwünschten Problemen (Dosierung, Infektionen etc.) einhergehen kann.

In einer bevorzugten Ausführungsform wird *in vivo* ein maximaler Serumspiegel an Wirkstoff innerhalb von höchstens 120 Minuten, bevorzugter innerhalb von höchstens 90 Minuten, noch bevorzugter innerhalb von höchstens 60 Minuten nach Verabreichung der pharmazeutischen Formulierung erreicht.

Weiter ist es bevorzugt, dass weitere Bestandteile der pharmazeutischen Formulierung ausgewählt sind aus Weichmachern und filmbildenden Polymeren. Ein bevorzugter Weichmacher ist beispielsweise Glycerol. Ein bevorzugtes filmbildendes Polymer ist beispielsweise Hydroxypropylmethylcellulose.

Zudem ist es bevorzugt, dass ein Anteil an Tensid in der pharmazeutischen Formulierung 0,001 bis 0,5 Gew.-%, noch bevorzugter 0,01 bis 0,3 Gew.-%,noch bevorzugter 0,025 bis 0,1 Gew.-%, bezogen auf die Gesamtzusammensetzung der pharmazeutischen Formulierung, beträgt.

Ferner ist es bevorzugt, dass ein Anteil an mikronisierendem Polymer in der pharmazeutischen Formulierung 0,1 bis 2 Gew.-%, noch bevorzugter 0,5 bis 1 Gew.-%, bezogen auf die Gesamtzusammensetzung der pharmazeutischen Formulierung, beträgt.

Es ist bevorzugt, dass ein Anteil an Wirkstoff in der pharmazeutischen Formulierung 0,5 bis 5 Gew.-%, noch bevorzugter 2 bis 3 Gew.-%, bezogen auf die Gesamtzusammensetzung der pharmazeutischen Formulierung, beträgt.

Ferner ist es bevorzugt, dass ein Anteil an filmbildendem Polymer in der pharmazeutischen Formulierung 2 bis 30 Gew.-%, noch bevorzugter 7 bis 17 Gew.-%, bezogen auf die Gesamtzusammensetzung der pharmazeutischen Formulierung, beträgt.

Zudem ist es bevorzugt, dass ein Anteil an Weichmacher in der pharmazeutischen Formulierung 1 bis 20 Gew.-%, noch bevorzugter 2 bis 8 Gew.-%, bezogen auf die Gesamtzusammensetzung der pharmazeutischen Formulierung, beträgt.

Es ist bevorzugt, dass ein Anteil an Wasser in der pharmazeutischen Formulierung 20 bis 95 Gew.-%, noch bevorzugter 40 bis 85 Gew.-%, bezogen auf die Gesamtzusammensetzung der pharmazeutischen Formulierung, beträgt.

Gegenstand der Erfindung ist ferner ein Verfahren zum Herstellen einer pharmazeutischen Formulierung wie oben zuvor beschrieben, wobei ein Zerkleinern des Tadalafil-Wirkstoffes zumindest zusammen mit dem mikronisierenden Polymer (PVP und/oder KVA) und einem Tensid (beispielsweise SDS) erfolgt. Ein Zerkleinern des Wirkstoffes zumindest zusammen mit dem mikronisierenden Polymer und dem Tensid meint im Rahmen der Erfindung dabei, dass das mikronisierende Polymer und das Tensid bereits während des Verfahrensschrittes der Zerkleinerung neben dem Wirkstoff in der Zusammensetzung vorhanden sein müssen. Weitere Bestandteile der pharmazeutischen Formulierung können bereits während der Zerkleinerung des Wirkstoffes in der pharmazeutischen Formulierung vorhanden sein oder können zu einem späteren Zeitpunkt nach der Zerkleinerung zugegeben werden.

Wie bereits zuvor beschrieben ist es bevorzugt, dass neben den mikronisierenden Polymeren Polyvinylpyrrolidon und/oder Vinylpyrrolidon-Vinylacetat-Copolymer, auch Hydroxypropylmethylcellulose, Hydroxypropylcellulose, Methylcellulose und Gemischen hiervon umfasst sind.

Weiter bevorzugt ist, dass das Tensid ein anionisches Tensid ist. Noch bevorzugter ist das anionische Tensid ausgewählt aus Alkylsulfaten, Alkylsulfonaten, Arylsulfaten, Arylsulfonaten und Gemischen hiervon. In einer besonders bevorzugten Ausführungsform ist das anionische Tensid Natriumdodecylsulfat.

Bevorzugt ist ferner, dass die mittlere Partikelgröße des Wirkstoffes nach dem Zerkleinern in einem Bereich von 8 bis 500 nm, bevorzugter in einem Bereich von 10 bis 390 nm, noch bevorzugter in einem Bereich von 100 bis 390 nm, am meisten bevorzugt in einem Bereich von 200 bis 350 nm liegt.

Bevorzugt wird das Zerkleinern für eine Dauer von 100 bis 260 Minuten, bevorzugt für eine Dauer von 140 bis 180 Minuten durchgeführt.

Zudem ist es bevorzugt, dass das Zerkleinern ein Vermahlen, noch bevorzugter ein Nassvermahlen ist.

Weiter ist es bevorzugt, dass das Zerkleinern in einer Kugelmühle, bevorzugter in einer Rührwerkskugelmühle erfolgt und wobei das Vermahlen in der Rührwerkskugelmühe vorzugsweise mit einer Rührerumfangsgeschwindigkeit von mehr als 4 m/s, vorzugsweise 5-15 m/s, weiter vorzugsweise 7-11 m/s, besonders bevorzugt 9 m/s erfolgt.

Den Komponenten a), b) und c) der pharmazeutischen Formulierung können während und/oder nach dem gemeinsamen Zerkleinern weitere Komponenten zugesetzt werden. Beispielsweise können

i) den Komponenten a), b) und c) nach dem gemeinsamen Zerkleinern in einer Rührwerkskugelmühle weitere Komponenten zur Herstellung eines Films in die Rührwerkskugelmühle hinzugegeben werden, wobei besagte weitere Komponenten vorzugsweise wasserlösliche Cellulosederivate umfassen,
ii) die daraus resultierende Gesamtmischung in der Rührwerkskugelmühle wird homogenisiert und anschließend
iii) das erhaltene Homogenat als Beschichtungsmasse auf einem Film aufgebracht oder selbst zu einem Film verarbeitet.

Der vorgenannte Homogenisierungsschritt ii) kann in der Rührwerkskugelmühe mit einer Rührerumfangsgeschwindigkeit von mehr als 2 m/s, vorzugsweise 3-12 m/s, weiter vorzugsweise 4-8 m/s, besonders bevorzugt 6 m/s erfolgen.

Ferner ist es bevorzugt, dass ein Anteil an Tensid in der pharmazeutischen Formulierung 0,001 bis 0,5 Gew.-%, noch bevorzugter 0,01 bis 0,3 Gew.-%,noch bevorzugter 0,025 bis 0,1 Gew.-%, bezogen auf die Gesamtzusammensetzung der pharmazeutischen Formulierung, beträgt.

Ein weiterer Gegenstand der Erfindung ist eine pharmazeutische Formulierung, erhältlich durch das erfindungsgemäße Verfahren zum Herstellen einer pharmazeutischen Formulierung wie zuvor oben beschrieben.

Ein weiterer Gegenstand der Erfindung ist ein Arzneimittel umfassend die erfindungsgemäße pharmazeutische Formulierung oder die pharmazeutische Formulierung, erhältlich durch das erfindungsgemäße Verfahren. Das Arzneimittel kann wie bereits zuvor für die pharmazeutische Formulierung beschrieben formuliert sein, beispielsweise als Schmelz-, Lutsch- oder Bukkal-Tablette, eine Kapsel, die eine darin enthaltende wässerige Suspension, Lösung, Tinktur, Creme, Paste, Lotion, Salbe, Gel im Mundraum freisetzt bzw. diese Formulierungen unverkapselt selbst, ein Aerososol oder als Film. Besonders bevorzugt ist die Formulierung als Film. Denn die erfindungsgemäße pharmazeutische Formulierung lässt sich durch ihre oben genannten Eigenschaften homogen auf einem Film verteilen. Zudem wird bei der oralen Verabreichung oder oralen Aufnahme eines Films eine besonders schnelle Wirkstoffresorption durch die Mundschleimhaut ermöglicht.

Ein weiterer Gegenstand der Erfindung ist das erfindungsgemäße Arzneimittel zur Verwendung bei der Behandlung einer sexuellen Dysfunktion, vorzugsweise einer erektilen Dysfunktion.

Zudem ist es bevorzugt, dass *in vivo* ein maximaler Serumspiegel an Wirkstoff ($t_{max}$) innerhalb von höchstens 120 Minuten, vorzugsweise innerhalb von höchstens 90 Minuten, weiter bevorzugt innerhalb von höchstens 60 Minuten, besonders bevorzugt innerhalb von 45 Minuten nach Verabreichung der pharmazeutischen Formulierung erreicht wird.

Die Erfindung wird nun anhand vorteilhafter Ausführungsformen unter Bezugnahme auf die beigefügten Zeichnungen erläutert. Es zeigt:

Fig. 1: Versuchsergebnisse der Zerkleinerung von Tadalafil mit Additiven.

Fig. 2: die Veränderung der Partikelgröße während der Zerkleinerung von Tadalafil.

Fig.3: Freisetzungsprofile von Tadalafil-Filmzubereitungen im Vergleich zum Handelspräparat Cialis® (5 mg).

Fig. 4-7: Diagramme (y-Achse - Konzentration (ng/ml); x-Achse - Zeit (min))mit Versuchsergebnissen der Cross-Over-Studie zur Plasmaspiegelbestimmung.

1. Untersuchung zur Zerkleinerung von Tadalafil mit Additiven

Die Untersuchungen zur Formulierung von Tadalafil gegen Partikelagglomeration während der Zerkleinerung erfolgten in einer Planetenkugelmühle (PM400, Retsch). Es wurden insgesamt 11 verschiedenen sterische und elektrosterische Formulierungen getestet. Hierzu wurden Mahlräume aus Zirkonoxid mit einem Mahlraumvolumen von 1 ml verwendet. Die unterschiedlich formulierten Suspensionen wurden mit Yttrium-stabilisierten Zirkonoxid ($ZrO_2$, Sigmund Lindner) Mahlkörpen mit einem Durchmesser von $d_{50,MK}$= 475 $\mu$m zerkleinert, wobei der Mahlkörperfüllgrad $\varphi_{MK}$ = 0,5 betrug. Die Tadalafilpartikel wurden in der Mühle für 2 Stunden bei einer Sonnenradgeschwindigkeit von $v_{Sonne}$ = 400 U $min^{-1}$ beansprucht. Anschließend wurden die Suspensionen mittels dynamischer Lichtstreuung (Nanophox, SympaTec) vermessen. Das Prinzip der dynamische Lichtstreuung (DLS) wird für die Charakterisierung von Partikelgrößen eingesetzt und basiert auf der Erfassung der Streulichtintensität von sich thermisch bewegenden Partikeln. Hierzu wurden jeweils 200 $\mu$l der Suspension in 1 ml destilliertem Wasser verdünnt. Die verdünnte Suspension wurde dann in eine Acrylglasküvette überführt und zum Vermessen bei Raumtemperatur und unter Normaldruck in den Strahlengang des Geräts gestellt.

Die für die Formulierungen verwendeten Additive, deren Abkürzungen sowie Lieferanten der Additive sind in Tabelle 1 dargestellt.

Tabelle 1: Getestete Additive

| **Additiv** | **Abkürzung** | **Lieferant** |
|---|---|---|
| Polyvinylpyrrolidone | PVP | Sigma Aldrich |
| Vinylpyrrolidon-Vinylacetat-Copolymer (Kollidon®VA64) | KVA | BASF |
| Hydroxypropylcellulose | HPC | Sigma Aldrich |
| Hydroxypropylmethylcellulose | HPMC | ShinEtsu |
| Methylcellulose | MC | Sigma Aldrich |
| Natriumdodecylsulfat | SDS | Sigma Aldrich |

Es wurden Formulierungen aus den oben genannten Additiven in destilliertem Wasser hergestellt. Die Tadalafil-Konzentration in der Suspension betrug stets $c_m$ = 0,05. Es wurde eine Additivkonzentration der sterisch stabilisierten Formulierungen von $c_{Add}$ = 0,4 gewählt (alle Additivanteile beziehen sich auf die Feststoffkonzentration). Bei den elektrosterisch stabilisierten Proben betrug der Polymeranteil $c_{Poly}$ = 0,3 und der Tensidanteil $c_{Tensid}$ = 0,1. Die Versuchsergebnisse sind in Figur 1 dargestellt. Allen Ergebnissen liegt eine Doppelbestimmung zugrunde.

Als Referenz wurde eine Zerkleinerung ohne Additiv durchgeführt, die deutlich macht, dass eine mittlere Partikelgröße unter $x_{50}$ = 7 $\mu$m nicht ohne Partikelstabilisierung erreicht werden kann. Die Ergebnisse der Zerkleinerung zeigen, dass mit fast allen gewählten Additiven eine Partikelgröße von unter $x_{50}$ = 700 nm in einer Zerkleinerungszeit von 2 Stunden erreicht werden kann. Mit den Cellulosen HPC und MC können mittlere Partikelgrößen von unter $x_{50}$ = 410 nm erreicht werden. Die besten Ergebnisse werden jedoch erst durch die zusätzliche Zugabe von Tensid erreicht, die einer elektrostatischen Aufladung entgegenwirkt und so zu einer weiteren Stabilisierung der Tadalafilpartikel führt. Aufgrund der, mittels Tensid zusätzlich aufgebrachten Ladung auf das Partikel, erfolgt eine noch effizientere Stabilisierung gegen Agglomeration während der Zerkleinerung. Die am besten geeignete Formulierung, mit einer erreichten mittleren Partikelgröße $x_{50}$ = 230 nm, ist die Kombination aus dem Polymer PVP und dem Tensid SDS.

2. Zerkleinerung von Tadalafil

Die Zerkleinerung des pharmazeutischen Wirkstoffes Tadalafil ($x_{50}$ = 6,4 μm) erfolgte in einer Rührwerkskugelmühle (MiniCer, Netzsch) mit zwei verschiedenen Formulierungen, umfassend das Polymer PVP oder KVA und das Tensid SDS. Die Handelsnamen der Additive sowie die Lieferanten sind der Tabelle 1 zu entnehmen.

Um eine Wirkstoffbeladung von 10 mg in einem 6 $cm^2$ orodispersiblen Film (ODF) zu realisieren wurde ein Feststoffgehalt ($m_{total}$ = 500 g) von $c_m$ = 0,033 in der Suspension vorgelegt. Eine Polymerkonzentration $c_{Poly}$ = 0,25 und eine Tensidkonzentration $c_{SDS}$ = 0,025 (beide bezogen auf den Feststoffgehalt in der Suspension) wurden zur Stabilisierung der Suspension gegen Agglomeration während der Zerkleinerung hinzugegeben. Die während der Zerkleinerung verwendeten Prozessparameter sind in Tabelle 2 dargestellt.

Tabelle 2: Prozessparameter während der Zerkleinerung in der Rührwerkskugelmühle

| | | |
|---|---|---|
| Allgemeine Parameter | Mahlkörpermaterial<br>Mahlkörpergröße<br>Mahlkörpefüllgrad | Zirkonoxid<br>$d_{MK}$ = 325 μm<br>$\varphi_{MK}$ = 0,8 |
| Zerkleinerung | Rührerumfangsgeschwindigkeit<br>Zerkleinerungszeit | $v_t$ = 9 m/s<br>$t_{zer}$= 140 min bzw. 180 min |
| Homogenisierung | Rührerumfangsgeschwindigkeit<br>Homogenisierungszeit | $v_t$ =6 m/s<br>$t_{Homo}$ = 20 min |

Die Veränderung der mittleren Partikelgröße während des Zerkleinerungsprozesses wurde auf den spezifischen Energieeintrag der Mühle bezogen (Figur 2). Für beide Formulierungen wird eine schnelle Zunahme der Partikelfeinheit innerhalb der ersten Minuten der Zerkleinerung von einer Ausgangspartikelgröße $x_{50}$ = 6,4 μm auf unter $x_{50}$ = 430 nm beobachtet. Mit zunehmender Zerkleinerungszeit sinkt die mittlere Partikelgröße bis zu einem Energieeintrag von $E_m$ = 55000 kJ $kg^{-1}$ für die Formulierung mit KVA und SDS auf 267 nm bzw. für die Formulierung mit PVP und SDS auf 342 nm. Anschließend wird eine leichte Partikelagglomeration beobachtet, was darauf zurück zu führen ist, dass die Stabilisierung der Partikel aufgrund der neu geschaffenen Oberflächen nicht mehr ausreichend ist. Es wird deutlich, dass die Formulierung mit KVA und SDS für die Tadalafilpartikel in wässriger Suspension besonders geeignet ist, da kleinere mittlere Partikelgrößen bei der Zerkleinerung mit gleichem Energieeintrag erreicht werden können.

Nach Abschluss der Zerkleinerung wurden 115 ml der Suspension aus der Mühle für weitere Versuche entnommen. Die restliche Suspension wurde direkt in der Mühle zu einer Beschichtungsmasse verarbeitet. Hierfür wurde die Methode von Steiner et al., "Efficient production of nanoparticle-loaded orodispersible films by process integration in a stirred media mill," International Journal of Pharmaceutics, 2016, vol. 511, S. 804-813, verwendet. Zu der Suspension wurde das filmbindende Polymer HPMC (Pharmacoat 606, ShinEtsu) direkt in die Mühle gegeben ($c_{HPMC}$ = 0,15) und nach der Auflösung des Polymers für 20 min homogenisiert. Anschließend erfolgte die Zugabe des Weichmachers Glycerol ($c_{Gly}$ = 0,05) und eine erneute Homogenisierung der Beschichtungsmasse für weitere 5 min in der Mühle. Die Probe wurde dann aus der Mühle entnommen und luftdicht verschlossen.

3. Herstellung von Filmen

Materialien

Tabelle 3: Liste der verwendeten Materialien

| **Substanz** | **Abkürzung** | **Lieferant** |
|---|---|---|
| Tadalafil | | |
| Polyvinylpyrrolidone | PVP | Sigma Aldrich |

(fortgesetzt)

| Substanz | Abkürzung | Lieferant |
|---|---|---|
| Vinylpyrrolidon-Vinylacetat Copolymer (Kollidon®VA64) | KVA | BASF |
| Hydroxypropylmethylcellulose (Pharmacoat 606®) | HPMC | ShinEtsu |
| Natriumdodecylsulfat | SDS | Sigma Aldrich |
| Glycerol | Gly | Caelo |
| Destilliertes Wasser | | |

Filmherstellung

Dosis pro Film: 8,12 mg
Filmdicke (nass): 500 μm
Größe eines Films: 6 $cm^2$

Tabelle 4: Zusammensetzung der Filmzubereitungen

| Substanz | Tadalafil, KVA & SDS | Tadalafil, PVP& SDS |
|---|---|---|
| Tadalafil | 2,73% | 2,73% |
| PVP | - | 0, 679% |
| KVP | 0,679% | - |
| SDS | 0,068% | 0,068% |
| HPMC | 12,43% | 12,43% |
| Glycerol | 4,75% | 4,75% |
| Wasser | 79,33% | 79,33% |

a) Zerkleinerung des Wirkstoffes

Die Zerkleinerung des Wirkstoffs erfolgte in einer Rührwerkskugelmühle (MiniCer, Netzsch, Deutschland). Dazu wurden das der jeweiligen Formulierung entsprechende Polymer PVP oder KVA zur sterischen Stabilisierung und SDS zur elektrostatischen Stabilisierung in destilliertem Wasser unter Rühren gelöst. Anschließend wurde der Wirkstoff Tadalafil hinzugegeben. Die Suspension umfassend Tadalafil, KVA und SDS wurde für 140 min und die Suspension umfassend Tadalafil, PVP und SDS wurde für 180 min unter Verwendung von Zirkonoxid (325 μm; 80% Mahlkörperfüllgrad) mit einer Geschwindigkeit von 9 m/s nassvermahlen.

b) Herstellung der Filme

Nach der jeweiligen Zerkleinerung des Wirkstoffs wurden das filmbildende Polymer HPMC und der Weichmacher Glycerol den in der Rührwerkskugelmühle jeweils befindlichen Suspensionen zugesetzt. Die Mischungen wurden für weitere 20 min mit einer Geschwindigkeit von 6 m/s homogenisiert. Die Mischungen wurden für 12 Stunden langsam (50 rpm) gerührt, um Luftblasen zu entfernen. Die Filme wurden mit einer Rakelhöhe von 500 μm unter Verwendung einer automatischen Filmziehbank (Coatmaster500, Erichsen) auf einer Polyethylenterephthalatfolie mit einer Geschwindigkeit von 6 mm/s bei Raumtemperatur hergestellt. Nach der Trocknung der Filme für 12 Stunden bei Raumtemperatur, wurden die hergestellten Filme manuell in rechteckige Stücke (2x3 cm) geschnitten.

Analysemethoden

a) Partikelgrößenbestimmung

Die Partikelgrößenverteilung der Suspensionen wurde mittels dynamischer Lichtstreuung (Nanophox, SympaTec) bestimmt. Dazu wurden jeweils ca. 200 μl der Filmsuspension in 2 ml destillierten Wasser verdünnt und bei Raumtemperatur und unter Normaldruck vermessen.

b) Freisetzung

Die Freisetzungsversuche wurden in 900 ml destilliertem Wasser bei 37±0,5°C unter Verwendung der Blattrührerapparatur (DT700, Erweka, Apparatur 1 nach Ph.Eur.) bei einer Geschwindigkeit von 100 rpm durchgeführt. Dazu wurden die Filme mit doppelseitigem Klebeband auf einer Glasplatte (Ø 7 cm) befestigt, die so auf einen Vesselboden gelegt wurde, dass die Filme mittig unter dem Blattrührer positioniert waren. Im Fall der bekannten Cialis® Tabletten (5 mg) wurde auf die Befestigung an der Glasplatte verzichtet. Der Probenzug erfolgte manuell zu den festgelegten Zeitpunkten. Die Proben (4 ml) wurden mit einer 5 ml Einmalspritze (Soft-Ject® 5 ml) über eine Fritte (Porendurchmesser 100 μm) entnommen und über einen Spritzenvorsatzfilter (Puradisc® 25, Whatman, PVDF Membran, Ø 25 mm, Porendurchmesser 0,2 μm) in ein HPLC-Vial gefiltert, wobei die ersten 3 ml verworfen wurden. Die dem Vessel entnommene Flüssigkeit wurde mit 4 ml vortemperierten (37±0,5°C) destilliertem Wasser ergänzt. Nach jeweils 5 Probenentnahmen wurde der Filter ersetzt. Die Probenkonzentration wurde mittels HPLC-MS/MS bestimmt.

Ergebnisse

a) Partikelgrößenbestimmung

Die Ausgangspartikelgröße betrug 6,4 μm ( x50% (Q3)). Der X50(Q3) bedeutet die mittlere Partikelgröße (mediane Partikelgröße) ermittelt aus einer Summenverteilung Q3 basierend auf der Erfassung des Partikelvolumens unter Verwendung der folgenden Formel:

$$\text{Summenhäufigkeit Q3i} = \sum \frac{\Delta M_i}{M_{gesamt}} * 100$$

Die mittleren Partikelgrößen reduzierten sich während der Zerkleinerung bereits in den ersten Minuten auf eine Größe von unter 430 μm (x50% (Q3)). Die Partikelgröße von Tadalafil in Kombination mit KVA und SDS wurde auf 267 nm (x50% (Q3)) und von Tadalafil in Kombination mit PVP und SDS auf 342 nm (x50% (Q3)) reduziert (vgl. Fig. 2).

b) Freisetzung

Die Freisetzungen erfolgten in allen Fällen unter Non-Sink Bedingungen, d.h. die Sättigungskonzentration von Tadalafil im wässrigen Medium wurde überschritten, so dass eine gesättigte Lösung mit Niederschlag entstand. Die freigesetzte Menge an Tadalafil hängt demnach von der Löslichkeit des Wirkstoffs im Freisetzungsmedium (destilliertes Wasser) ab. In Figur 3 sind die Freisetzungskurven der beiden Filmzubereitungen im Vergleich zu den bekannten Cialis® Tabletten (5 mg) dargestellt. Es wird deutlich, dass sich die Freisetzungsgeschwindigkeiten der verschiedenen Formulierungen kaum unterscheiden, da es sich sowohl bei den Filmformulierungen als auch bei der Tablette um schnellzerfallende Arzneiformen handelt. Die Filmzubereitungen setzen den Wirkstoff Tadalafil etwas schneller frei (innerhalb der ersten 60 Minuten) als das Handelspräparat Cialis®. Es ist allerdings deutlich zu erkennen, dass die Filmzubereitungen deutlich höhere maximale Konzentrationen erreichen. Besonders ausgeprägt ist dies bei den Filmen umfassend Tadalafil, KVA und SDS. Die Ursache hierfür liegt in den deutlich verringerten Wirkstoffpartikelgrößen der Filme, die zu einer deutlich erhöhten allgemeinen Löslichkeit und zu einer höheren Lösungsgeschwindigkeit des Wirkstoffes führen.

Permeationsstudien an Probanden

a) Methodik

Einschlusskriterien zur Studie waren ein Alter über 18 Jahre und keine schwerwiegenden Begleiterkrankungen. Ausschlusskriterien zur Studie war der Nachweis von Tadalafil zum Zeitpunkt des Beginns der Studienphase (t0).

b) Studienmedikation Pharmakokinetik

Im Rahmen der Studien wurden die folgenden Präparate verwendet:

- Als Vergleich das Handelspräparat Cialis® von Lilly Pharma, 10 mg (übliche Dosis zur Behandlung der erektilen Dysfunktion)
- Film umfassend Tadalafil, KVA und SDS, Herstellung wie oben unter Filmherstellung beschrieben, 8 mg

- Film umfassen Tadalafil, PVP und SDS, Herstellung wie oben unter Filmherstellung beschrieben, 8 mg

c) Cross-Over Studie - Plasmaspiegelbestimmung

In einer Cross-Over-Studien können Zielparameter wie Behandlungsformen oder Plasmaspiegel miteinander verglichen werden. Dabei werden die untersuchten Mittel sequentiell den gleichen Probanden verabreicht. Verglichen mit herkömmlichen Studien mit parallelen Vergleichsgruppen haben Cross-Over-Studien den Vorteil, dass kleinere Unterschiede der Zielparameter (z.B. Plasmaspiegel) statistisch signifikant werden oder dass für den Nachweis eines signifikanten Unterschieds weniger Teilnehmer notwendig sind.

Bei einer Cross-Over-Studie ist der sogenannte Carry-Over-Effekt zu berücksichtigen, das heißt, das Hinüberwirken der Anwendungseffekte des ersten Mittels in die nächstfolgende Anwendungsphase. Deshalb bedarf es zwischen den Behandlungsphasen einer Pause, in der keine Anwendung stattfindet, damit eine Wirkung des ersten Mittels nicht mehr gegeben ist. Zu Beginn jeder Studienphase bzw. zum Zeitpunkt $t_0$ wird daher der Wirkstoff-Plasmaspiegel bestimmt und die Studie wird gewertet, wenn kein Wirkstoff nachweisbar ist, also $t_0 = 0$.

Ergebnisse:

Die Figuren 4 bis 7 zeigen Diagramme (y-Achse - Konzentration (ng/ml); x-Achse - Zeit (min)), in denen die Versuchsergebnisse der Cross-Over-Studie zur Plasmaspiegelbestimmung dargestellt sind.

Die Figuren 4 und 5 zeigen dabei die Mittelwerte für die Serumspiegel von 4 unterschiedlichen Probanden. Die Linie A repräsentiert den Serumspiegel nach einer Einnahme von 8 mg Tadalafil, formuliert in der Filmtablette (ODF) mit dem mikronisierenden Polymer PVP und dem Tensid SDS, während diese Linie in Fig. 4 so eigenständig benannt und der $t_{max}$-Wert von <40 min zur schnelleren Übersicht hinzugefügt wurde. In Fig. 4 wurde in gleicher Weise der Serumspiegel nach einer Einnahme von 8 mg Tadalafil, formuliert in der Filmtablette (ODF) mit dem mikronisierenden Polymer KVA und dem Tensid SDS benannt und ebenfalls der $t_{max}$-Wert von <40 min zur schnelleren Übersicht hinzugefügt. Die Linie B repräsentiert den Serumspiegel nach einer Einnahme von 10 mg Tadalafil, formuliert in einer handelsüblichen Tablette Cialis® (Lilly Pharma) in Fig. 5, während diese Linie in Fig. 4 so eigenständig benannt und der $t_{max}$-Wert von >240 min zur schnelleren Übersicht hinzufügt wurde.

Die Figuren 6 und 7 zeigen Einzelwerte für die einzelnen Probanden DS und JB. Die Linien A repräsentieren den Serumspiegel nach einer Einnahme von 8 mg Tadalafil, formuliert in der Filmtablette (ODF) mit dem mikronisierenden Polymer PVP und dem Tensid SDS. Die Linien B repräsentieren den Serumspiegel nach einer Einnahme von 10 mg Tadalafil, formuliert in einer handelsüblichen Tablette Cialis® (Lilly Pharma).

Fazit

In den Ausführungsbeispielen konnte gezeigt werden, dass durch die Zugabe einer Kombination von mikronisierendem Polymer und Tensid eine Stabilisierung der zerkleinerten Wirkstoffpartikel mit einer mittleren Partikelgröße von weit unter 500 nm, insbesondere unter 390 nm unter Verwendung einer Rührwerkskugelmühle problemlos möglich ist. Die inline Herstellung der Filmmasse in der Rührwerkskugelmühle gemäß der Methode nach Steiner et al., "Efficient production of nanoparticle-loaded orodispersible films by process integration in a stirred media mill," International Journal of Pharmaceutics, 2016, vol. 511, S. 804-813, ist einfach umsetzbar. Die Filme lassen sich auf diese Weise problemlos herstellen. Entsprechende Versuche zur Wirkstofffreisetzung zeigen, dass sowohl die allgemeine Löslichkeit des Wirkstoffs Tadalafil in wässrigen Medien als auch die Lösungsgeschwindigkeit durch die Zerkleinerung der Wirkstoffpartikel deutlich gesteigert werden kann. In weiterführenden Untersuchungen konnte zudem gezeigt werden, dass dieses eine Steigerung der pharmakologischen Effizienz durch einen schnelleren Wirkeintritt und eine erhöhte Bioverfügbarkeit zur Folge hat wenn PVP bzw. KVA Verwendung finden.

## Patentansprüche

1. Pharmazeutische bukkale, sublinguale, gingivale oder intranasale Formulierung umfassend die folgenden Komponenten:

   a) Tadalafil oder dessen Salz als Wirkstoff, wobei die mittlere Partikelgröße des Wirkstoffes in besagter Formulierung in einem Bereich von 8 bis 500 nm liegt, vermessen mittels dynamischer Lichtstreuung (DLS),
   b) ein mikronisierendes Polymer, wobei besagtes Polymer Polyvinylpyrrolidon (PVP) und/oder Vinylpyrrolidon-Vinylacetat-Copolymer (KVA) ist, und
   c) ein Tensid;

erhältlich durch ein Verfahren, in dem ein Zerkleinern von a) Tadalafil oder dessen Salz als Wirkstoff zumindest zusammen mit dem Polymer b) und dem Tensid c) erfolgt.

2. Pharmazeutische Formulierung nach Anspruch 1, **dadurch gekennzeichnet, dass** die mittlere Partikelgröße des Wirkstoffes gemäß Komponente a) in einem Bereich von 10 bis 390 nm, noch bevorzugter in einem Bereich von 100 bis 390 nm, am meisten bevorzugt in einem Bereich von 200 bis 350 nm liegt.

3. Pharmazeutische Formulierung nach Anspruch 1 oder 2, **dadurch gekennzeichnet, dass** besagte Formulierung neben dem Polymer gemäß Komponente b) mindestens ein weiteres Polymer umfasst, ausgewählt aus der Gruppe bestehend aus Hydroxypropylmethylcellulose, Hydroxypropylcellulose, Methylcellulose und Gemischen hiervon.

4. Pharmazeutische Formulierung nach einem der Ansprüche 1 bis 3, **dadurch gekennzeichnet, dass** das Tensid gemäß Komponente c) ein anionisches Tensid, vorzugsweise ein anionisches Tensid ausgewählt aus Alkylsulfaten, Alkylsulfonaten, Arylsulfaten, Arylsulfonaten und Gemischen hiervon, besonders bevorzugt Natriumdodecylsulfat (SDS), ist.

5. Pharmazeutische Formulierung nach einem der Ansprüche 1 bis 4, **dadurch gekennzeichnet, dass**

- besagte Polymer gemäß Komponente b) PVP und besagtes Tensid gemäß Komponente c) SDS sind, oder
- besagtes Polymer gemäß Komponente b) KVA und besagtes Tensid gemäß Komponente c) SDS sind, oder
- besagtes Polymer gemäß Komponente b) ein Gemisch aus PVP und KVA und besagtes Tensid gemäß Komponente c) SDS sind.

6. Pharmazeutische Formulierung nach einem der Ansprüche 1 bis 5, **dadurch gekennzeichnet, dass** die pharmazeutische Formulierung ausgewählt ist aus der Gruppe bestehend aus

i) einem Film,
ii) einem Aerosol,
iii) einer wässrigen Suspension, Lösung, Tinktur, Creme, Paste, Lotion, Salbe, einem Gel, oder diese Formulierungen im Mundraum freisetzende Kapsel,
iv) einer Schmelz-, Lutsch- oder Bukkal-Tablette,

wobei vorgenannte Formulierungen vorzugsweise mukoadhäsive Formulierungen sind.

7. Pharmazeutische Formulierung nach einem der Anspruch 1-6, wobei der Wirkstoff in der Formulierung über die Mucosa der Mundhöhle oder Nase in den Blutkreislauf zur systemischen Wirkung verabreichbar ist.

8. Verfahren zum Herstellen einer pharmazeutischen Formulierung nach einem der vorangehenden Ansprüche, wobei ein Zerkleinern des Wirkstoffes gemäß Komponente a) zumindest zusammen mit dem Polymer gemäß Komponente b) und dem Tensid gemäß Komponente c) erfolgt.

9. Verfahren nach Anspruch 8, **dadurch gekennzeichnet, dass** das Zerkleinern für eine Dauer von 100 bis 260 Minuten, bevorzugt von 140 bis 180 Minuten erfolgt.

10. Verfahren nach Anspruch 8 oder 9, **dadurch gekennzeichnet, dass** das Zerkleinern ein Vermahlen, bevorzugt ein Nassvermahlen ist, und wobei vorzugsweise das Vermahlen in einer Rührwerkskugelmühe mit einer Rührerumfangsgeschwindigkeit von mehr als 4 m/s, vorzugsweise 5-15 m/s, weiter vorzugsweise 7-11 m/s, besonders bevorzugt 9 m/s erfolgt.

11. Verfahren nach einem der Ansprüche 8-10, **dadurch gekennzeichnet, dass** den Komponenten a), b) und c) während und/oder nach dem gemeinsamen Zerkleinern weitere Komponenten zugesetzt werden.

12. Verfahren nach einem der Ansprüche 8-10, **dadurch gekennzeichnet, dass**

i) den Komponenten a), b) und c) nach dem gemeinsamen Zerkleinern in einer Rührwerkskugelmühle weitere Komponenten zur Herstellung eines Films in die Rührwerkskugelmühle hinzugegeben werden, wobei besagte weitere Komponenten vorzugsweise wasserlösliche Cellulosederivate umfassen,
ii) die daraus resultierende Gesamtmischung in der Rührwerkskugelmühle homogenisiert wird und anschließend

iii) das erhaltene Homogenat als Beschichtungsmasse auf einem Film aufgebracht oder selbst zu einem Film verarbeitet wird.

**13.** Verfahren nach Anspruch 12, **dadurch gekennzeichnet, dass** der Homogenisierungsschritt ii) in der Rührwerkskugelmühe mit einer Rührerumfangsgeschwindigkeit von mehr als 2 m/s, vorzugsweise 3-12 m/s, weiter vorzugsweise 4-8 m/s, besonders bevorzugt 6 m/s erfolgt.

**14.** Arzneimittel umfassend die pharmazeutische Formulierung nach einem der Ansprüche 1 bis 7 oder die pharmazeutische Formulierung, erhältlich durch das Verfahren nach einem der Ansprüche 8 bis 13, zur Verwendung bei der Behandlung einer sexuellen Dysfunktion, vorzugsweise einer erektilen Dysfunktion.

**15.** Arzneimittel zur Verwendung nach Anspruch 14, wobei der maximale Serumspiegel an Wirkstoff ($t_{max}$) innerhalb von höchstens 120 Minuten, vorzugsweise innerhalb von höchstens 90 Minuten, weiter bevorzugt innerhalb von höchstens 60 Minuten, besonders bevorzugt innerhalb von 45 Minuten nach Verabreichung der pharmazeutischen Formulierung erreicht wird.

## Claims

**1.** Pharmaceutical buccal, sublingual, gingival or intranasal formulation comprising the following components:

a) tadalafil or salt thereof as active substance, the average particle size of the active substance in said formulation being within a range from 8 to 500 nm, measured by dynamic light scattering (DLS),
b) a micronizing polymer, said polymer being polyvinylpyrrolidone (PVP) and/or vinylpyrrolidone-vinyl acetate copolymer (KVA), and
c) a surfactant,

obtainable by a method wherein a) tadalafil or salt thereof as active substance is comminuted at least together with the polymer b) and the surfactant c).

**2.** Pharmaceutical formulation according to Claim 1, **characterized in that** the average particle size of the active substance according to component a) is within a range from 10 to 390 nm, more preferably within a range from 100 to 390 nm, most preferably within a range from 200 to 350 nm.

**3.** Pharmaceutical formulation according to Claim 1 or 2, **characterized in that** said formulation comprises, in addition to the polymer according to component b), at least one further polymer selected from hydroxypropyl methylcellulose, hydroxypropyl cellulose, methylcellulose, and mixtures thereof.

**4.** Pharmaceutical formulation according to any of Claims 1 to 3, **characterized in that** the surfactant according to component c) is an anionic surfactant, preferably an anionic surfactant selected from alkyl sulfates, alkyl sulfonates, aryl sulfates, aryl sulfonates, and mixtures thereof, more preferably sodium dodecyl sulfate (SDS).

**5.** Pharmaceutical formulation according to any of Claims 1 to 4, **characterized in that**

- said polymer according to component b) are PVP and said surfactant according to component c) are SDS, or
- said polymer according to component b) are KVA and said surfactant according to component c) are SDS, or
- said polymer according to component b) are a mixture of PVP and KVA and said surfactant according to component c) are SDS.

**6.** Pharmaceutical formulation according to any of Claims 1 to 5, **characterized in that** the pharmaceutical formulation is selected from the group consisting of

i) a film,
ii) an aerosol,
iii) an aqueous suspension, solution, tincture, cream, paste, lotion, ointment, gel, or capsule releasing these formulations in the oral cavity,
iv) an orodispersible tablet, lozenge or buccal tablet,

the abovementioned formulations preferably being mucoadhesive formulations.

7. Pharmaceutical formulation according to any of Claims 1-6, wherein the active substance in the formulation can be administered into the bloodstream for a systemic action via the mucosa of the oral cavity or nose.

8. Method for producing a pharmaceutical formulation according to any of the preceding claims, wherein comminution of the active substance according to component a) is carried out at least together with the polymer according to component b) and the surfactant according to component c).

9. Method according to Claim 8, **characterized in that** comminution is carried out for a period of 100 to 260 minutes, preferably for a period of 140 to 180 minutes.

10. Method according to Claim 8 or 9, **characterized in that** comminution is a milling process, more preferably wet milling, and wherein milling preferably takes place in a stirring ball mill at a peripheral stirrer speed of more than 4 m/s, preferably 5-15 m/s, more preferably 7-11 m/s, particularly preferably 9 m/s.

11. Method according to any of Claims 8-10, **characterized in that** further components are added to components a), b), and c) during and/or after the combined comminution thereof.

12. Method according to any of Claims 8-10, **characterized in that**

    i) after combined comminution of components a), b), and c) in a stirring ball mill, further components to produce a film are added to the stirring ball mill, said further components preferably comprising water-soluble cellulose derivatives,
    ii) the resulting total mixture in the stirring ball mill is homogenized, and then
    iii) the homogenate obtained is applied to a film as a coating compound or is itself processed into a film.

13. Method according to Claim 12, **characterized in that** the homogenization step ii) is carried out in the stirring ball mill at a peripheral stirrer speed of more than 2 m/s, preferably 3-12 m/s, more preferably 4-8 m/s, particularly preferably 6 m/s.

14. Medicament comprising the pharmaceutical formulation according to any of Claims 1 to 7 or the pharmaceutical formulation obtainable by the method according to any of Claims 8 to 13, for use in the treatment of sexual dysfunction, preferably erectile dysfunction.

15. Medicament for use according to Claim 14, wherein the maximum serum active substance concentration ($t_{max}$) is reached within not more than 120 minutes, preferably within not more than 90 minutes, more preferably within not more than 60 minutes, particularly preferably within not more than 45 minutes, after administration of the pharmaceutical formulation.

## Revendications

1. Formulation pharmaceutique buccale, sublinguale, gingivale ou intranasale, comprenant les composants suivants :

    a) du tadalafil ou son sel en tant que principe actif, la taille de particule moyenne du principe actif dans ladite formulation se situant dans une plage de 8 à 500 nm, mesurée par diffusion dynamique de la lumière (DLS),
    b) un polymère micronisant, ledit polymère étant la polyvinylpyrrolidone (PVP) et/ou un copolymère de vinylpyrrolidone-acétate de vinyle (KVA), et
    c) un tensioactif ;

    pouvant être obtenue par un procédé dans lequel un broyage de a) tadalafil ou de son sel en tant que principe actif est effectué au moins conjointement avec le polymère b) et le tensioactif c).

2. Formulation pharmaceutique selon la revendication 1, **caractérisée en ce que** la taille de particule moyenne du principe actif selon le composant a) se situe dans une plage de 10 à 390 nm, de manière encore plus préférée dans une plage de 100 à 390 nm, de manière la plus préférée dans une plage de 200 à 350 nm.

**3.** Formulation pharmaceutique selon la revendication 1 ou 2, **caractérisée en ce que** ladite formulation comprend, outre le polymère selon le composant b), au moins un autre polymère choisi dans le groupe constitué par l'hydroxypropylméthylcellulose, l'hydroxypropylcellulose, la méthylcellulose et leurs mélanges.

**4.** Formulation pharmaceutique selon l'une quelconque des revendications 1 à 3, **caractérisée en ce que** le tensioactif selon le composant c) est un tensioactif anionique, de préférence un tensioactif anionique choisi parmi les alkylsulfates, les alkylsulfonates, les arylsulfates, les arylsulfonates et leurs mélanges, de manière particulièrement préférée le dodécylsulfate de sodium (SDS).

**5.** Formulation pharmaceutique selon l'une quelconque des revendications 1 à 4, **caractérisée en ce que**

- ledit polymère selon le composant b) est le PVP et ledit tensioactif selon le composant c) est le SDS, ou
- ledit polymère selon composant b) est le KVA et ledit tensioactif selon le composant c) est le SDS, ou
- ledit polymère selon le composant b) est un mélange de PVP et de KVA et ledit tensioactif selon le composant c) est le SDS.

**6.** Formulation pharmaceutique selon l'une quelconque des revendications 1 à 5, **caractérisée en ce que** la formulation pharmaceutique est choisie dans le groupe constitué par

i) un film,
ii) un aérosol,
iii) une suspension aqueuse, une solution, une teinture, une crème, une pâte, une lotion, un onguent, un gel, ou une capsule libérant ces formulations dans la bouche,
iv) un comprimé à fondre, à sucer ou buccal,

les formulations susmentionnées étant de préférence des formulations mucoadhésives.

**7.** Formulation pharmaceutique selon l'une quelconque des revendications 1 à 6, dans laquelle le principe actif dans la formulation peut être administré via la muqueuse de la cavité buccale ou du nez dans la circulation sanguine pour un effet systémique.

**8.** Procédé de préparation d'une formulation pharmaceutique selon l'une quelconque des revendications précédentes, dans lequel un broyage du principe actif selon le composant a) est effectué au moins conjointement avec le polymère selon le composant b) et le tensioactif selon le composant c).

**9.** Procédé selon la revendication 8, **caractérisé en ce que** le broyage est effectué pendant une durée de 100 à 260 minutes, de préférence de 140 à 180 minutes.

**10.** Procédé selon la revendication 8 ou 9, **caractérisé en ce que** le broyage est une pulvérisation, de préférence une pulvérisation humide, et dans lequel, de préférence, la pulvérisation est effectuée dans un broyeur à billes à agitateur avec une vitesse périphérique d'agitateur supérieure à 4 m/s, de préférence de 5 à 15 m/s, de préférence encore de 7 à 11 m/s, de manière davantage préférée de 9 m/s.

**11.** Procédé selon l'une des revendications 8 à 10, **caractérisé en ce que** d'autres composants sont ajoutés aux composants a), b) et c) pendant et/ou après le broyage commun.

**12.** Procédé selon l'une quelconque des revendications 8 à 10, **caractérisé en ce que**

i) on ajoute aux composants a), b) et c), après le broyage commun dans un broyeur à billes à agitateur, d'autres composants pour produire un film dans le broyeur à billes à agitateur, lesdits autres composants comprenant de préférence des dérivés de cellulose hydrosolubles,
ii) le mélange total qui en résulte est homogénéisé dans le broyeur à billes à agitateur, puis
iii) le produit homogénéisé obtenu est appliqué sur un film en tant que masse de revêtement ou est lui-même transformé en un film.

**13.** Procédé selon la revendication 12, **caractérisé en ce que** l'étape d'homogénéisation ii) est effectuée dans le broyeur à billes à agitateur à une vitesse périphérique de l'agitateur supérieure à 2 m/s, de préférence de 3 à 12 m/s, de préférence encore de 4 à 8 m/s, de manière particulièrement préférée de 6 m/s.

**14.** Médicament comprenant la formulation pharmaceutique selon l'une quelconque des revendications 1 à 7 ou la formulation pharmaceutique pouvant être obtenue par le procédé selon l'une quelconque des revendications 8 à 13, pour une utilisation dans le traitement d'un dysfonctionnement sexuel, de préférence d'un dysfonctionnement érectile.

**15.** Médicament pour utilisation selon la revendication 14, dans lequel le taux sérique maximal de principe actif ($t_{max}$) est atteint dans un délai d'au plus 120 minutes, de préférence dans un délai d'au plus 90 minutes, de préférence encore dans un délai d'au plus 60 minutes, de manière particulièrement préférée dans un délai d'au plus 45 minutes après l'administration de la formulation pharmaceutique.

Fig.1

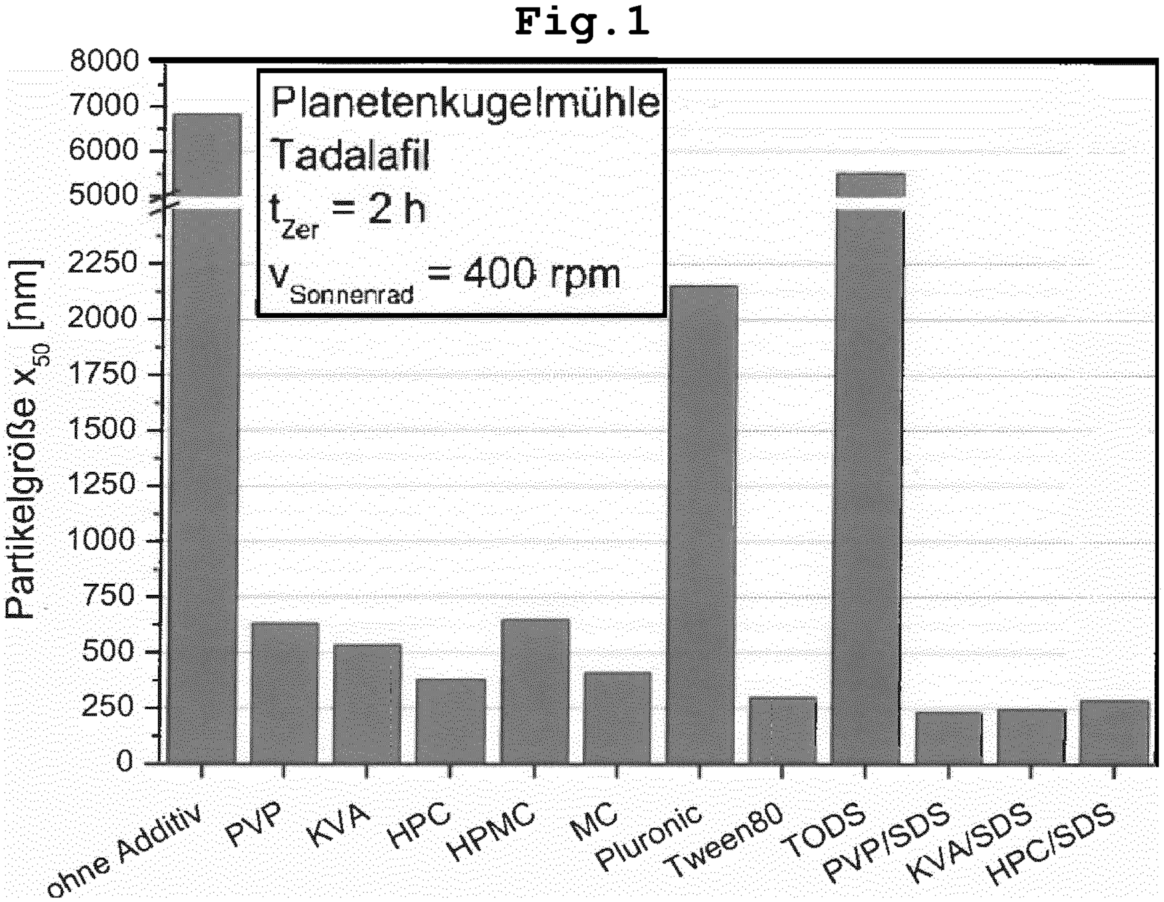
Planetenkugelmühle
Tadalafil
$t_{Zer}$ = 2 h
$v_{Sonnenrad}$ = 400 rpm
Partikelgröße $x_{50}$ [nm]
8000
7000
6000
5000
2250
2000
1750
1500
1250
1000
750
500
250
0
ohne Additiv
PVP
KVA
HPC
HPMC
MC
Pluronic
Tween80
TODS
PVP/SDS
KVA/SDS
HPC/SDS

Fig.2

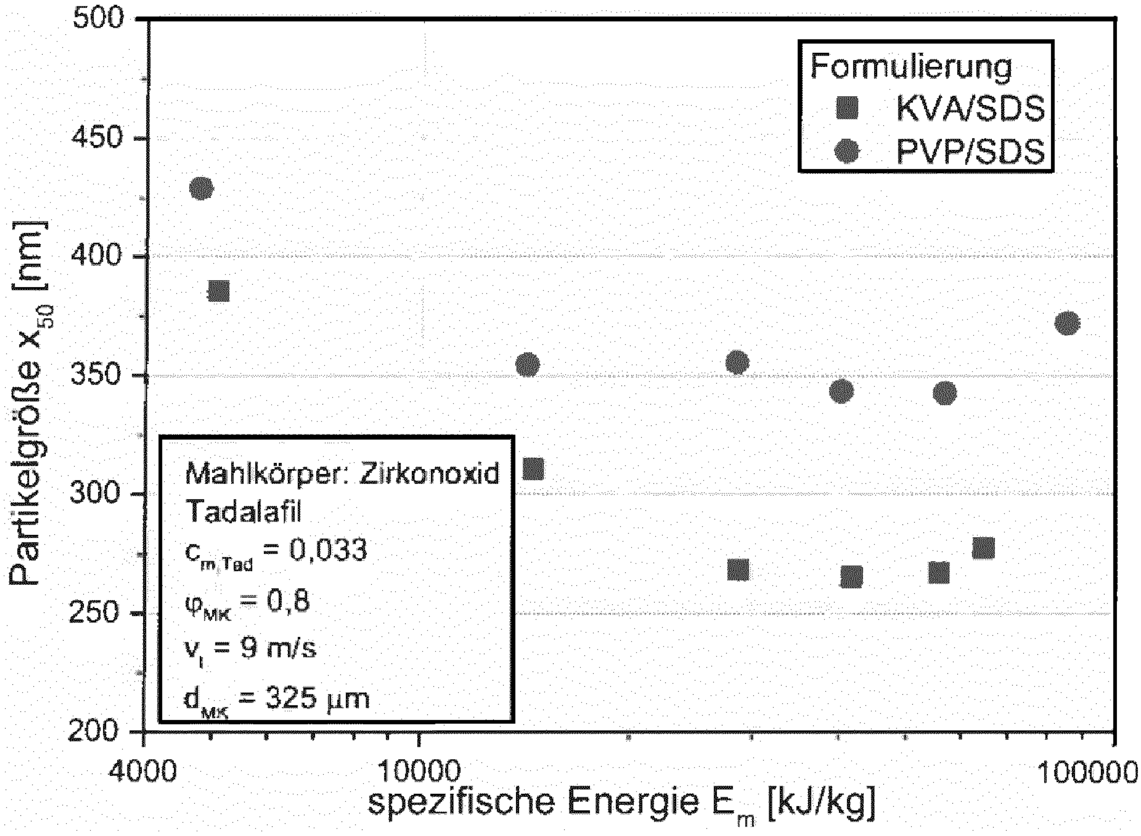
Formulierung
KVA/SDS
PVP/SDS
Mahlkörper: Zirkonoxid
Tadalafil
$c_{m,Tad}$ = 0,033
$\varphi_{MK}$ = 0,8
$v_t$ = 9 m/s
$d_{MK}$ = 325 µm
Partikelgröße $x_{50}$ [nm]
500
450
400
350
300
250
200
4000
10000
100000
spezifische Energie $E_m$ [kJ/kg]

Fig.3

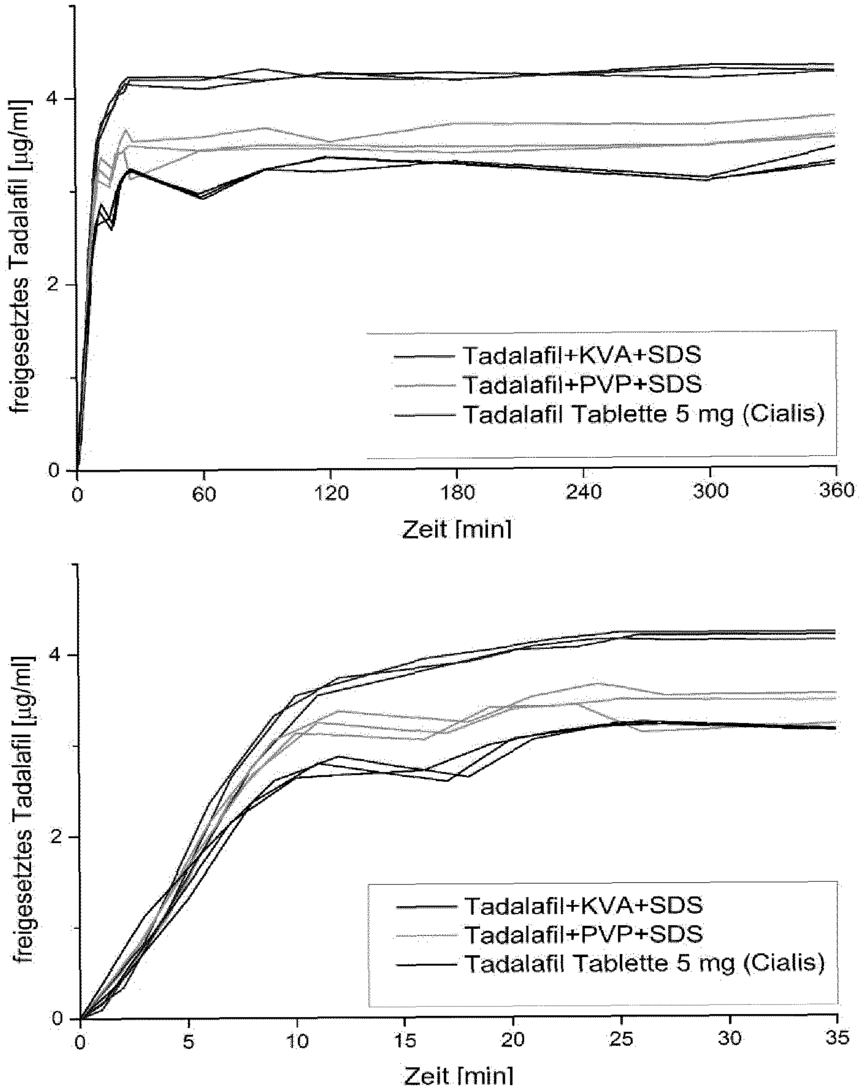
freigesetztes Tadalafil [µg/ml]
0
2
4
0
60
120
180
240
300
360
Zeit [min]
Tadalafil+KVA+SDS
Tadalafil+PVP+SDS
Tadalafil Tablette 5 mg (Cialis)
freigesetztes Tadalafil [µg/ml]
0
2
4
0
5
10
15
20
25
30
35
Zeit [min]
Tadalafil+KVA+SDS
Tadalafil+PVP+SDS
Tadalafil Tablette 5 mg (Cialis)

Fig.4

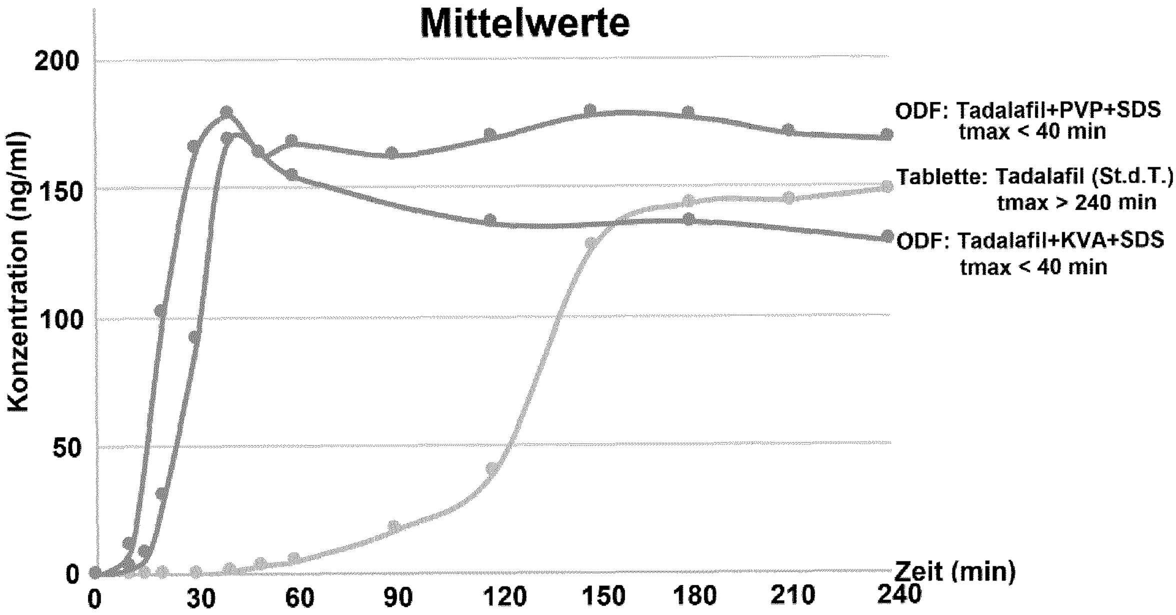
Mittelwerte
Konzentration (ng/ml)
0
50
100
150
200
0
30
60
90
120
150
180
210
240
Zeit (min)
ODF: Tadalafil+PVP+SDS
tmax < 40 min
Tablette: Tadalafil (St.d.T.)
tmax > 240 min
ODF: Tadalafil+KVA+SDS
tmax < 40 min

Fig.5

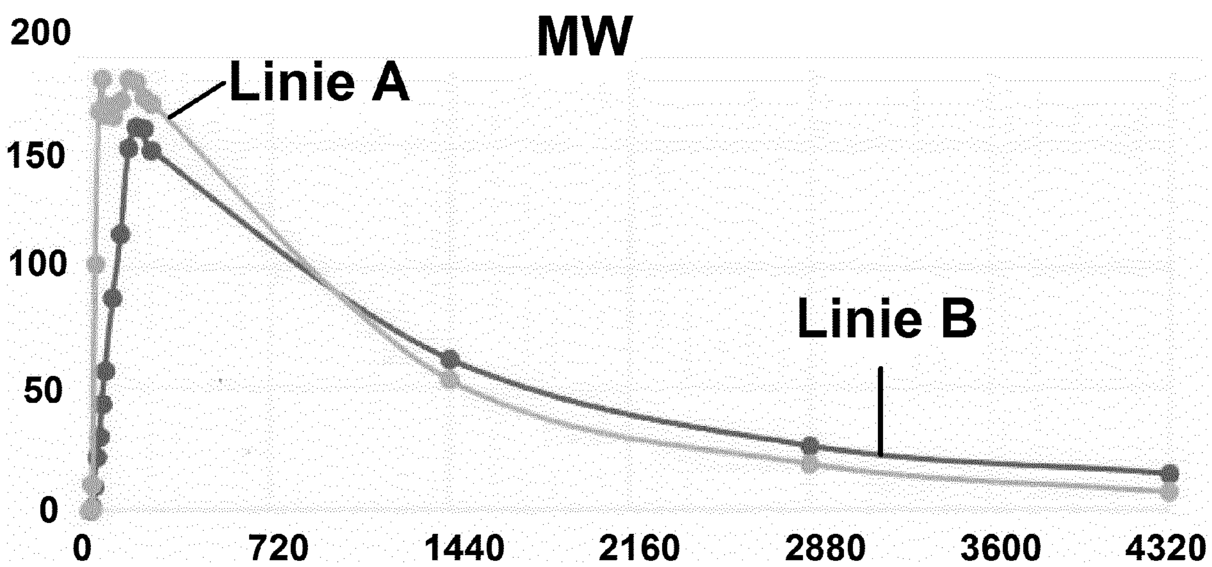
MW
Linie A
Linie B
200
150
100
50
0
0
720
1440
2160
2880
3600
4320

Fig.6

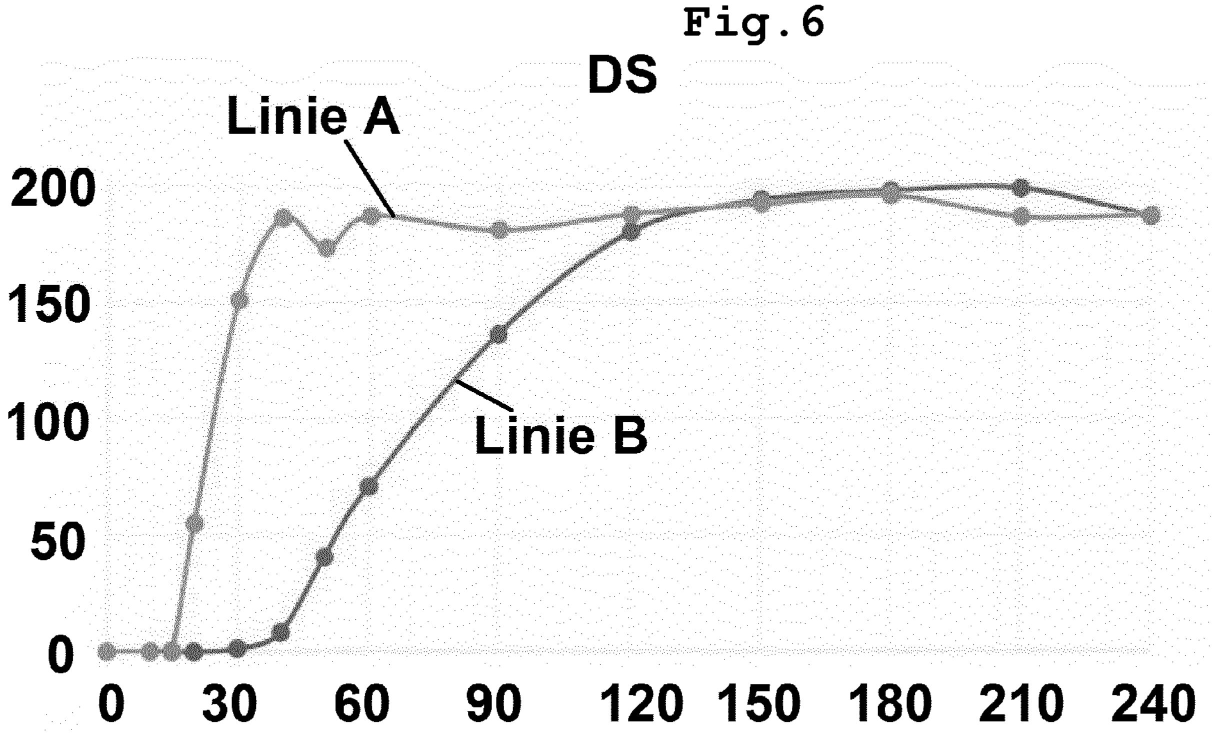
DS
Linie A
Linie B
200
150
100
50
0
0
30
60
90
120
150
180
210
240

Fig.7

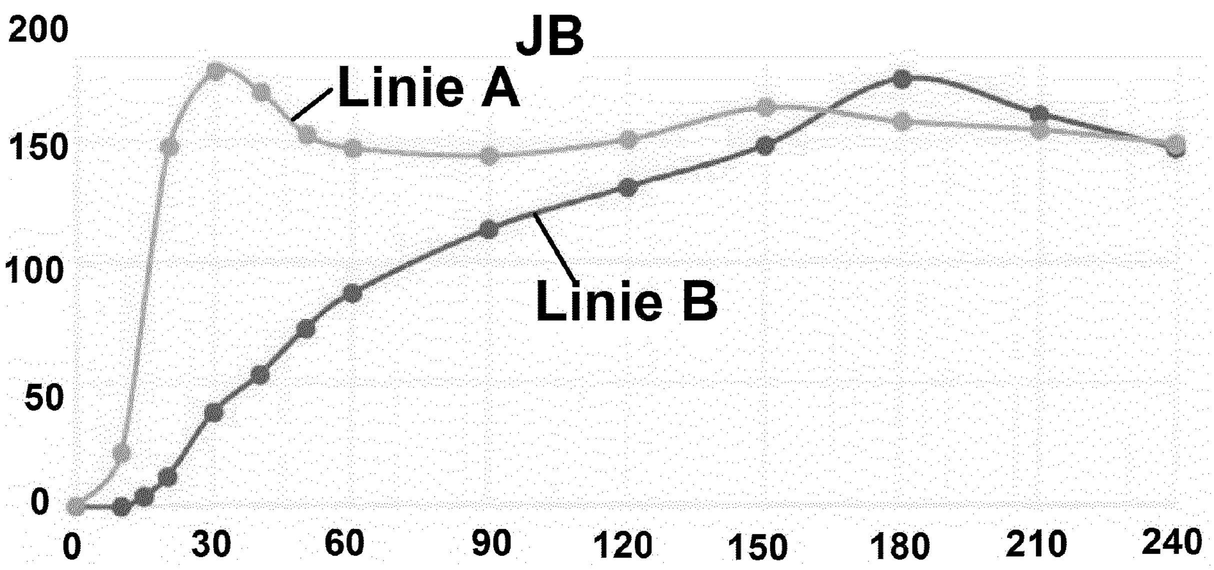

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20150359735 A1
- US 20150359735 A


### In der Beschreibung aufgeführte Nicht-Patentliteratur


- **STEINER et al.** Efficient production of nanoparticle-loaded orodispersible films by process integration in a stirred media mill. *International Journal of Pharmaceutics*, 2016, vol. 511, 804-813